(19) **Europäisches Patentamt / European Patent Office / Office européen des brevets**

(11) **EP 1 147 396 B1**

(12) **EUROPÄISCHE PATENTSCHRIFT**

(45) Veröffentlichungstag und Bekanntmachung des Hinweises auf die Patenterteilung:
**14.03.2007   Patentblatt 2007/11**

(21) Anmeldenummer: **00982984.7**

(22) Anmeldetag: **11.10.2000**

(51) Int Cl.:
*G01N 21/35* (2006.01)

(86) Internationale Anmeldenummer:
**PCT/DE2000/003572**

(87) Internationale Veröffentlichungsnummer:
**WO 2001/027595 (19.04.2001 Gazette 2001/16)**

(54) **GASQUALITÄTSBESTIMMUNG**

DETERMINATION OF THE QUALITY OF A GAS

PROCEDE POUR DETERMINER LA QUALITE DE GAZ

(84) Benannte Vertragsstaaten:
**AT BE CH CY DE DK ES FI FR GB GR IE IT LI LU MC NL PT SE**

(30) Priorität: **14.10.1999   DE 19949439**

(43) Veröffentlichungstag der Anmeldung:
**24.10.2001   Patentblatt 2001/43**

(73) Patentinhaber: **Elster-Instromet Systems GmbH 44357 Dortmund (DE)**

(72) Erfinder: **KASTNER, Joachim 44139 Dortmund (DE)**

(74) Vertreter: **Schneider, Uwe
Patentanwalt
Holbeinstrasse 27
59423 Unna (DE)**

(56) Entgegenhaltungen:
**EP-A- 0 882 977          US-A- 3 950 101
US-A- 4 594 510**

EP 1 147 396 B1

**Beschreibung**

Die Erfindung betrifft ein Verfahren zur photometrischen Bestimmung der Gasqualität, insbesondere von Brenngasen, gemäß Oberbegriff des Anspruches 1.

Zur Erfassung der Gasbeschaffenheit zum Beispiel in Verteilungsnetzen für Erdgas oder dgl. werden schon seit langem Einrichtungen zur Erfassung der Beschaffenheit des jeweils durchgeleiteten Gases, sog. Gasbeschaffenheitsmeßgeräte benutzt. Erdgas unterliegt als Naturprodukt je nach Herkunft und durch Mischung entsprechenden Schwankungen bezüglich seiner Zusammensetzung, wobei die Zusammensetzung z.B. von Erdgas aus den verschiedenen Kohlenwasserstoffen aber wesentlich den Brennwert und daraus abgeleitete Größen bestimmt. Daher ist es für die Abrechnung der durch ein Gasversorgungsnetz durchgeleiteten Gasmenge und damit der entsprechenden Energiemenge von großer Wichtigkeit, die jeweilige Gasbeschaffenheit an der Einspeisestelle in das Erdgasnetz und der Abnahmestelle des Kunden genau zu erfassen und damit eine tatsächlich transportierte bzw. gelieferte Energiemenge zu bestimmen und abzurechnen. Somit kann dem Abnehmer des Gases auch bei sich unterscheidender Gasbeschaffenheit und einem entsprechend schwankendem Energiegehalt immer ein dem tatsächlich gelieferten Energiegehalt entsprechender Preis in Rechnung gestellt werden. Umgekehrt bietet die Erfassung der Gasbeschaffenheit dem Abnehmer die Gewähr, nachprüfbar eine von ihm verlangte Qualität und damit geforderten Energiegehalt geliefert zu erhalten.

Die Erfassung der Gasbeschaffenheit erhält zusätzliche Bedeutung, da mit Fall des Durchleitungsmonopols der Erdgasversorger durch ein und dasselbe Versorgungsnetz Gase ganz unterschiedlicher Herkunft und damit auch unterschiedlicher Zusammensetzung durchgeleitet werden. Nur eine möglichst einfache und kostengünstige Erfassung der Gasbeschaffenheit durch entsprechend bereitgestellte kostengünstige Meßgeräte und Meßverfahren erlaubt dann eine nachvollziehbare und genaue Abrechnung.

Zur Bestimmung der Gasqualität müssen als maßgebliche Größen möglichst genau und auch Veränderungen der Gasqualität erfassend der volumetrische Normbrennwert $H_{v,n}$, die Normdichte $\rho n$ und die Kompressibilitätszahl $K$ bestimmt werden.

In der Praxis wird für die energetische Abrechnung zunächst mittels Durchflußmeßgeräten das transportierte Gasvolumen $V_b$ unter Betriebsbedingungen (Druck $p_b$, Temperatur $T_b$) gemessen. Bei Kenntnis der Gasbeschaffenheit läßt sich die Kompressibilitätszahl $K$ bestimmen, mit der das Gasvolumen $V_n$ unter Normbedingungen (Druck $p_n$, Temperatur $T_n$) berechnet wird.

$$V_n = \frac{p_b\, T_n}{p_n\, T_b}\, \frac{1}{K} V_b$$

Durch Multiplikation dieses Normvolumens mit dem volumetrischen Brennwert Hv,n unter Normbedingungen erhält man die transportierte Energiemenge Q:

$$Q = V_n H_{v,n}$$

Alternativ kann auch direkt das Betriebsvolumen $V_b$ mit dem Betriebsbrennwert $H_{v,b}$ multipliziert werden (Energiemeter).

Eine weitere wichtige Größe bei Erdgasanwendungen ist die thermische Leistungsabgabe von Gasbrennern; sie variiert ebenso mit der Gasbeschaffenheit und wird durch den sogenannten Wobbeindex $W_v$ charakterisiert: Gase mit gleichem Wobbeindex $W_v$ liefern die gleiche Wärmeleistung an einer Verbrennungsdüse. Zur Berechnung des Wobbeindexes $W_v$ ist die Normdichte $\rho_n$ des Gases erforderlich, aus der die relative Dichte bezüglich Luft bestimmt wird ($d_v = \rho_{Gas}/ \rho_{Luft}$)

$$W_v = \frac{H_v}{\sqrt{d_v}}$$

Daher kommt der Bestimmung des Normbrennwertes $H_{v,n}$ für die praktische Bestimmung der Gasqualität z.B.

zu Abrechnungszwecken zentrale Bedeutung zu.

Bisher finden verschiedene Einrichtungen zur Erfassung der Gasbeschaffenheit Anwendung. Man unterscheidet hierbei sog. direkte und sog. indirekte Verfahren. Bei den direkten Verfahren werden die zu bestimmenden Größen einzeln gemessen und hierzu das Gas meist auf Normbedingungen gebracht, wodurch teilweise aufwendige Gasvorbehandlungen erforderlich werden.

Am einfachsten läßt sich die Gasbeschaffenheit mittels sog. Kalorimeter bestimmen, bei denen mittels einer offenen Flamme Probengas verbrannt und aus der entstehenden und an ein Kühlmedium abgegebenen Wärmemenge und der daraufhin feststellbaren Temperaturerhöhung des Kühlmediums der Brennwert des verbrannten Gases ermittelt wird. Derartige Geräte benötigen eine komplizierte Mechanik zur Einstellung eines bestimmten Mengenverhältnisses von Gas, Verbrennungsluft und z.B. Kühlluft als Kühlmedium und sind daher teuer und fehleranfällig, zumal durch die offene Verbrennung erhöhte Sicherheitsanforderungen an die Geräte zu stellen sind. Auch muß die Wartung und Kalibrierung von Fachpersonal durchgeführt werden, darüber hinaus müssen die Kalorimeter in konditionierten Räumen eingesetzt werden. Daher liegen die Anschaffungs- und Betriebskosten derartiger Meßanordnungen sehr hoch.

Bei der Kalorimetrie durch katalytische Verbrennung (z. B. mit Pellistoren) wird das Probengas mit Luft gemischt und an den 400 bis 500˚C heißen Wendeln eines Katalysators verbrannt. Die Temperaturerhöhung des Katalysators ist dabei proportional zum Brennwert. Da dieses Verfahren auf einem empfindlichen Oberflächeneffekt basiert, ist es starken Driften unterworfen und erfordert häufige Kalibration mit Prüfgas. Die katalytischen Kalorimeter sind von allen hier beschriebenen Verfahren am günstigsten, allerdings eignen sie sich von ihrer Genauigkeit eher zur Kontrolle als zur Abrechnung.

Die direkte Messung der Betriebsdichte $\rho_b$ erfolgt zum einen mit Dichtewaagen, sehr aufwendigen Präzisionsgeräten, bei denen man den Auftrieb einer stickstoffgefüllten Kugel in Abhängigkeit der Dichte des umgebenden Mediums, hier des Probengases, mißt. Bei einem anderen Verfahren wird ein dünnwandiger Metallzylinder, der vom Probengas umströmt wird, in Schwingung versetzt. Die Dichte des umgebenden Gases bestimmt die Resonanzfrequenz des Zylinders, die als empfindliche Meßgröße erfaßt wird. Beide Verfahren sind für die Bestimmung der Normdichte sehr aufwendig, da sie die Einstellung des Normzustandes erfordern.

Die Kompressibilitätszahl K wird nun nicht direkt gemessen, sondern kann nach unterschiedlichen numerischen Standard-Berechnungsverfahren aus den direkt meßbaren Gasgrößen berechnet werden. Das eine Verfahren, das sog. GERG88-Verfahren (DVGW-Arbeitsblatt 486) benötigt dabei die in Tabelle 1 aufgeführten Eingangsgrößen. Der Stoffmengenanteil von $CO_2$ wird nach dem heutigen Stand der Technik durch ein nicht dispersives infrarotspektroskopisches Verfahren (NDIR) bestimmt, wobei das Gas in einen definierten Zustand nahe oder bei Normbedingung gebracht werden muß. Der Stoffmengenanteil von $H_2$ ist praktisch nur bei der Betrachtung von Kokereigasen von Bedeutung und kann in den heute in Europa verteilten typischen Erdgasen praktisch vernachlässigt werden. Die Kompressibilitäts-Zahl K läßt sich mit der GERG88-Gleichung bei genügender Genauigkeit der Eingangsgrößen auf $10^{-3}$ bestimmen.

Tabelle 1: Eingangsgrößen des GERG88-Verfahrens

| $P_b$ | Betriebsdruck |
|---|---|
| $T_b$ | Betriebstemperatur |
| $\rho_n$ | Dichte im Normzustand |
| $H_{v,n}$ | Volumetrischer Brennwert im Normzustand |
| $XCO_2$ | Stoffmengenanteil $CO_2$ |
| $XH_2$ | Stoffmengenanteil $H_2$ |

Das andere Verfahren zur Bestimmung des Realgasverhaltens erfolgt nach der AGA8-92DC-Gleichung (ISO 12213-2:1997 (E)). Dieses Verfahren benötigt als Eingangswerte die Stoffmengenanteile von 21 führenden Gaskomponenten (Tabelle 2) und erreicht ebenso eine Genauigkeit von $10^{-3}$.

Tabelle 2: Eingangsgrößen der AGA8-92DC-Gleichung

| Methan | $CO_2$ |
|---|---|
| Ethan | $N_2$ |
| Propan | $H_2S$ |
| Isobutan | He |
| n-Butan | $H_2O$ |

(fortgesetzt)

| Isopentan | $O_2$ |
|-----------|-------|
| n-Pentan | Ar |
| n-Hexan | $H_2$ |
| n-Heptan | CO |
| n-Oktan | Druck |
| n-Nonan | Temperatur |
| n-Dekan | |

Der Stand der Technik umfaßt neben den direkten Meßtechniken auch die indirekte Gasbeschaffenheitsmessung mittels Gaschromatographie. Dabei wird ein definiertes Volumen des Probengases in einen definierten Zustand gebracht und von einem Trägergas, typischerweise Helium, durch ein System von gaschromatographischen Trennsäulen getragen. Aufgrund ihrer unterschiedlichen Retentionszeiten erreichen die einzelnen Gaskomponenten den nachgeschalteten Sensor, im allgemeinen ist dies ein Wärmeleitfähigkeitsdetektor, am Ende der Trennsäule zeitlich getrennt. Die Peakfläche des Sensorsignals kann nun als Stoffmenge interpretiert werden, wobei die Auswertung im Vergleich zu einem Referenzgas erfolgen muß, das in etwa ähnliche Zusammensetzung wie das Probengas haben muß. Der Nachteil der Gaschromatographie liegt in der aufwendigen Probenaufbereitung und Installation des Gesamtsystems, und in der aufwendigen Unterhaltung und Bedienung durch geschultes Personal. Aus den Stoffmengenanteilen der einzelnen Gaskomponenten, wie sie die Gaschromatographie liefert, lassen sich dann alle relevanten Gasgrößen berechnen. Für die Realisierung derartiger indirekter Messungen durch Chromatographie werden automatisch arbeitende Prozeßchromatographen mit Wärmeleitfähigkeitsdetektoren eingesetzt. Diese Geräte messen normalerweise elf Komponenten des Erdgases ($N_2$, $CO_2$, $CH_4$, $C_2H_6$, $C_3H_8$, $C_4H_{10}$, $C_5H_{12}$, $C_6$+, usw.). Als Trägergas wird Helium verwendet, wobei dessen leichte Flüchtigkeit in der Praxis oft zur vorzeitigen Entleerung der Trägergasflasche führt und daher kurze Wartungszyklen eines derartigen Gasbeschaffenheitsmeßgerätes bedingt. Als Kalibriergas wird ein Gas gewählt, das dem zu messenden Erdgas ähnlich ist. Derartige Chromatographensysteme führen ohne Unterbrechung Meßzyklen aus, damit Änderungen der Gasbeschaffenheit sofort erfaßt werden. Dieses führt zu einem hohen Verbrauch an Trägergas und Kalibriergas und hat zudem zur Folge, daß Wartungen des Gerätes in relativ kurzen Abständen durchgeführt werden müssen.

Es ist ebenfalls bekannt, mit üblichen Infrarot-Gasanalysatoren die Zusammensetzung eines Gases zu bestimmen. Derartige im mittleren Infrarot oder nahen Infrarot arbeitende Analysatoren bieten jedoch nicht die für eine Brennwertbestimmung erforderlichen hohen Genauigkeits- und insbesondere Stabilitätsanforderungen unter den hier erforderlichen Meßbedingungen. Auch ist neben der eigentlichen Messung an dem Probengas immer eine parallel laufende Referenzmessung erforderlich, um zumindest die wesentlichsten Fehlereinflüsse kompensieren zu können. Als Meßergebnisse liefern die bekannten Infrarot-Gasanalysatoren überlagerte Frequenzspektren, die eine Rückbeziehung auf einzelne Komponenten eines untersuchten Gases stark erschweren, wenn nicht sogar unmöglich machen.

In der Literatur wurde auch ein infrarotspektroskopisches Verfahren zur Gasanalyse beschrieben ("Optical BTU Sensor Development", Gas Research Institute GRI-93/0083), das mittels sog. Multivariater Analyse (MVA) der Nahinfrarotspektren von Gasen die volumetrische Stoffmengenkonzentration der kohlenstoffhaltigen Komponenten des Gases und damit den volumetrischen Brennwert unter Betriebsbedingungen ermittelt. Dieses Verfahren liefert jedoch nicht den Brennwert $H_{v,n}$ unter Normbedingungen und nicht die Normdichte $\rho_n$ und den Stoffmengenanteil von $CO_2$, so daß es nicht zur Bestimmung der Kompressibilitäts-Zahl K und damit zur vollständigen Gasqualitätsbestimmung geeignet ist. Bei der Bestimmung des Brennwertes mittels vorbekannter photometrischer Methoden werden zudem geringere Ansprüche an die notwendige apparative Ausstattung zur Durchführung der Verfahren gestellt, wobei ein Vorteil in der Geschwindigkeit der Erfassung der Absorptionsspektren des Erdgases im nahen oder mittleren infraroten Spektralbereich liegt. Das gesamte Absorptionsspektrum des Erdgases setzt sich dabei aus der Summe der Einzelspektren der im Gas vorliegenden Komponenten additiv zusammen und kann daher gemessen und mit Hilfe geeigneter Spektrenanalyseverfahren analysiert werden. Hierbei ist der ermittelte Anteil einer Extinktion einer Komponente an dem gesamten Spektrum des Erdgases im wesentlichen dem Konzentrationsanteil dieser Komponente im Prüfgas gleich (sog. Beer-Lambert'sche Gesetz). Mit Kenntnis des Brennwertes der jeweiligen Komponente kann dann der Brennwert des gesamten Gasgemisches als Summenwert berechnet werden. Problematisch an diesem Verfahren der Spektrenanalyse ist jedoch die starke Überlappung von Absorptionsbanden unterschiedlicher Komponenten, die häufig zu ungenauen Ergebnissen führen und darüber hinaus einen hohen Rechenaufwand benötigen.

Ein weiteres infrarotspektroskopisches Verfahren nach der DE 198 38 301 A1 arbeitet als direkte Spektralauswertung (DSA) mit einer Spektralfunktion, mit der das Gasspektrum gefaltet wird. Das Verfahren erlaubt die Bestimmung des volumetrischen Brennwertes $H_{v,b}$ unter Betriebsbedingungen direkt aus dem Spektrum. Hierzu wird ausgenutzt, daß bei der Gasverbrennung die jeweils erzeugte Reaktionswärme auf der Verbrennung von C-H-Bindungen beruht und eine dabei erzeugte Wärmemenge von der vorliegenden Bindungsenergie abhängt. Dies wird dadurch ausgenutzt, daß die Schwingungen der C-H-Bindungen, die eine zueinander gleiche, bestimmte Bindungsenergie aufweisen und bei einer Verbrennung die gleiche Wärmemenge erzeugen, bei einer zugeordneten Wellenlänge in Wechselwirkung mit einer elektromagnetischen Strahlung treten. Hierdurch ist durch wellenlängen-aufgelöstes Messen und einer wellenlängen-abhängigen Gewichtung der Wechselwirkungsgrade dieser Schwingungen die Berechnung des Brennwertes $H_{v,b}$ des Gases möglich, ohne daß eine Identifizierung einzelner Gaskomponenten erforderlich ist. Es wird dort darüber hinaus eine Vorrichtung zur Durchführung eines derartigen Verfahrens vorgeschlagen, die auf die spezifischen Erfordernisse des Meßverfahrens abgestimmt ist und eine gewichtete Aufsummierung der Wechselwirkungsgrade ermöglicht. Mit diesem Verfahren kann zwar der Brennwert $H_{v,b}$ eines Gasgemisches unter Betriebsbedingungen ermittelt werden, die anderen zur Gasqualitätsmessung benötigten Größen lassen sich jedoch meßtechnisch hiermit nicht erfassen.

Es ist weiterhin ein Verfahren nach der DE 199 00 129.4 A1 bekannt, bei dem mit einem optisch-spektroskopischen Verfahren die Betriebsdichte eines Probengases bestimmt wird. Dieses Verfahren geht dabei von der Überlegung aus, daß jede Bindung der IR-aktiven Gaskomponenten zur Extinktion beiträgt, wobei andererseits mit jeder Bindung die Masse der anhängigen Atome verknüpft ist. Auf diese Weise ist die Masse aller IR-aktiven Atome durch Absorptionsbeiträge im Spektrum repräsentiert. Die Schwingungsfrequenz jeder Bindung und damit ihre spektrale Lage hängt von der reduzierten Masse der Bindungspartner ab. Damit enthält das Spektrum in seinem Betrag und seiner spektralen Verteilung Informationen zur Bestimmung der Dichte des Gases. Der Zusammenhang zwischen dem Spektrum und der Masse im Meßvolumen und damit mit der Betriebsdichte des Gases wird durch eine spektrale Gewichtungsfunktion beschrieben, die als spektrale Dichte bezeichnet werden kann. Die Massebeiträge der IR-inaktiven Komponenten werden über die Zustandsgrößen und einen geeigneten Ansatz zum Realgasverhalten berechnet. Bei einem derartigen Verfahren sind mehrfache Iterationen erforderlich, für die gewisse Annahmen hinsichtlich der Anfangsbedingungen getroffen werden müssen, die möglicherweise problematisch hinsichtlich der Konvergenz der Iteration sein können.

Aus der EP 0 882 977 A1, der US-PS 3 950 101 und der US-PS 4 594 510 sind Verfahren zur Erfassung des Brennwertes von Gasgemischen mittels spektroskopischer Messverfahren bekannt, wobei in der EP 0 882 977 A1 eine Messung parallel in mehreren Spektralbereichen erfolgt.

Es ist daher Aufgabe der vorliegenden Erfindung, ein Verfahren für die Bestimmung der Gasqualität vorzuschlagen, bei dem für die Gasqualität maßgebliche Größen anhand einer spektroskopischen Erfassung der Gasbeschaffenheit sowie entsprechenden Auswertungen bestimmt werden können.

Die Lösung der erfindungsgemäßen Aufgabe ergibt sich aus den kennzeichnenden Merkmalen des Anspruches 1 in Zusammenwirken mit den Merkmalen des Oberbe-griffes. Weitere vorteilhafte Ausgestaltungen der Erfindung ergeben sich aus den jeweiligen Unteransprüchen.

Die Erfindung geht aus von einem Verfahren zur Bestimmung der Gasqualität eines Probengases, insbesondere eines Brenngases, bei dem unter Betriebsbedingungen mittels spektroskopischer Meßverfahren ein Transmissionsspektrum des Probengases bestimmt wird. Ein derartiges gattungsgemäßes Verfahren wird dadurch weitergebildet, daß in einem ersten Schritt in einer Zustandsmessung zumindest die zugehörigen Werte des Druckes p und der Temperatur T des Probengases ermittelt werden, wonach in einem weiteren Schritt in einer Anzahl von ausgewählten Spektralbereichen durch Integration von Werten des Transmissionsspektrums des Probengases ein Spektralvektor $\vec{S}$ gebildet wird, der als Komponenten die Werte der Integrale über die ausgewählten Spektralbereiche aufweist und charakteristisch für die Zusammensetzung des Probengases im Betriebszustand ist. In einem weiteren Schritt wird zur Bestimmung einer im Hinblick auf die Gasqualität zu bestimmenden physikalischen Größe der jeweilige Spektralvektor $\vec{S}$ skalar mit einem Faktorenvektor $\vec{V}$ multipliziert, der durch Kalibrationsmessungen von Spektralvektoren $\vec{S}$ an Kalibriergasen bekannter Zusammensetzungen und unter bekannten Zustandsbedingungen ermittelt wurde, wobei je nach der im Hinblick auf die Gasqualität zu bestimmenden physikalischen Größe ein zugehöriger Faktorenvektor $\vec{V}$ herangezogen wird. Hierbei erfolgt die Bestimmung der im Hinblick auf die Gasqualität zu bestimmenden physikalischen Größe direkt unter Einbeziehung des Zustandes des Probengases durch Auswertung des Spektrums und ohne die bisher notwendigen Iterationsverfahren. Durch die Verknüpfung der aus der Zustandsmessung des Probengases gewonnen Daten für den Druck p und die Temperatur T mit dem Spektralvektor $\vec{S}$, der aus dem Transmissionsspektrum gewonnen wird, lassen sich insbesondere auch die Betriebsgrößen des Probengases, insbesondere Brennwert H und Dichte $\rho$ auf technisch besser nutzbare Normgrößen umwerten. Der Spektralvektor $\vec{S}$, der aus dem Transmissionsspektrum bestimmt wird, wird hierbei durch die Integration der Meßwerte der Extinktion des Transmissionsspektrums bestimmt, wobei diese Integrale nur in ausgewählten Spektralbereichen bestimmt werden, die auf üblicherweise in den zu untersuchenden Probengasen vorkommenden Komponenten abgestimmt werden können. Somit ist der Spektralvektor $\bar{S}$ charakteristisch für die Zusammensetzung des Probengases in dem jeweils vorliegenden Betriebszustand. Da der Betriebszustand des Probengases einen nicht unerheblichen Einfluß auf das Transmissionsspektrum des Probengases ausübt, wird aufgrund der Bestimmung des Transmissionsspektrums im Betriebszustand des Probengases dieser Einflußfaktor durch die sog. Faktorenvektoren $\vec{V}$ berücksichtigt, die durch Kalibrationsmessungen von Spektralvektoren $\bar{S}$ an definierten Kalibriergasen bestimmt werden, von denen die Zusammensetzung und damit das Soll-Transmissionsspektrum bekannt ist. Diese Faktorenvektoren $\vec{V}$ werden ebenfalls unter gleichzeitiger Messung der jeweils vorliegenden Zustandsbedingungen ermittelt, so daß der Einfluß von Temperatur T und Druck p auf das Transmissionsspektrum des Kalibriergases bekannt ist. Diese Faktorenvektoren $\vec{V}$ können dann durch die einfach ausführbare und ohne Iterationsschritte auskommende skalare Multiplikation mit dem Spektralvektor $\bar{S}$ durch einfache mathematische Operationen ausgeführt werden, wobei je nach der im Hinblick auf die Gasqualität zu bestimmende physikalische Größe ein zugehöriger Faktorenvektor $\vec{V}$ aus den Kalibrationsmessungen herangezogen wird. Somit läßt sich eine hohe Genauigkeit des erfindungsgemäßen Verfahrens auch über eine breite Meßspanne des Zustandes des Probengases erreichen, wobei insbesondere auch der Einfluß der Zustandsgrößen kompensiert werden kann.

Eine erste vorteilhafte Ausgestaltung sieht vor, daß die Faktorenvektoren $\vec{V}$ vorab in einer definierten Meßumgebung durch Kalibrationsmessungen bestimmt werden, die an Kalibriergasen bekannter Zusammensetzung und unter bekannten Zustandsbedingungen durchgeführt werden. Eine derartige definierte Meßumgebung bietet dabei die Möglichkeit, das der Berechnung der Faktorenvektoren $\vec{V}$ zugrundeliegende Transmissionsspektrum sowie die Zustandsgrößen Temperatur T und Druck p mit hoher Genauigkeit zu erfassen, die bei der Durchführung des Verfahrens im Feld nicht mit vertretbaren Kosten möglich ist. Die Faktorenvektoren $\vec{V}$ sind dabei hochgenaue Korrekturwerte für die Beeinflussung der Transmissionsmessung durch den Zustand des Probengases, wobei diese Faktorenvektoren räumlich und zeitlich getrennt von der Bestimmung der Spektralvektoren $\bar{S}$ ermittelt werden können.

Von besonderem Vorteil ist es, daß bei den Kalibrationsmessungen die Faktorenvektoren $\vec{V}$ in Form eines Kennfeldes unter Variation von Druck p und Temperatur T der verwendeten Kalibriergase bestimmt werden. Hierdurch ist es möglich, auch für breite Bereiche der Veränderung des Druckes p bzw. der Temperatur T die jeweils zugehörigen Faktorenvektoren $\vec{V}$ vorab zu bestimmen und für die skalare Multiplikation mit dem Spektralvektor $\bar{S}$ zur Verfügung zu stellen. Hierdurch wird zum einen die Veränderung des Transmissionsspektrums mit dem Druck p, die sogenannte Druckverbreiterung, als auch die Veränderung mit der Temperatur T berücksichtigt. Weiterhin können Variationen des Arbeitspunktes der Meßvorrichtung mit dem Zustand des Probengases kompensiert werden; diese treten zum Beispiel bei einem Spektrometer als Meßvorrichtung durch Variation der Flächenteilchendichte im Meßstrahl auf. Ebenfalls sind Realgaseffekte der Probengas kompensierbar, das sich üblicherweise nicht wie ein ideales Gas verhält, so daß zum Beispiel die Flächenteilchendichte im Meßstrahl nicht direkt proportional zum Quotienten aus Druck p und Temperatur T ist. Die Kalibration anhand der Kalibriergase erfolgt bei einer Reihe definierter Zustände in einem Zustandsfeld und für einen geeigneten Satz von Kalibriergasen, so daß man als Ergebnis für jede zu bestimmende Meßgröße ein Faktorenvektorkennfeld $\vec{V}$ (p.T) erhält, das durch die Zustandsgrößen Druck p und Temperatur T aufgespannt wird.

Von besonderem Vorteil ist es, wenn für jede der im Hinblick auf die Gasqualität zu bestimmenden physikalischen Größen aus dem Kennfeld für einen Zustand des Probengases ein zugehöriger Faktorenvektor $\vec{V}$ ermittelt wird. Bildlich gesprochen wird für jede der im Hinblick auf die Gasqualität zu bestimmenden physikalischen Größen aus dem Zustandskennfeld $\vec{V}$ (p,T) der Faktorenvektoren $\vec{V}$ eine Projektion auf die jeweilige Ebene der zu bestimmenden physikalischen Größen vorgenommen, so daß für jede zu bestimmende physikalische Größe ein speziell hieraus sich ergebender Faktorenvektor $\vec{V}_i$ bestimmt werden kann.

Aufgrund des Zusammenhanges zwischen den Zustandsgrößen Druck p und Temperatur T bei der Aufnahme des Spektralvektors $\bar{S}$ ist weiterhin von Vorteil, wenn zur Bildung des Skalarproduktes mit dem Spektralvektor $\bar{S}$

derjenige Faktorenvektor $\vec{V}$ aus dem Kennfeld ausgewählt wird, der den Zustandsbedingungen des Probengases bei der Aufnahme des Spektrum des Probengases und der im Hinblick auf die Gasqualität zu bestimmenden physikalischen Größe entspricht. Durch diese Zuordnung des Faktorenvektors $\vec{V}$ zu den am Probengas tatsächlich vorliegenden Zustandsgrößen Druck p und Temperatur T gelingt eine weitgehende Kompensation des Einflusses der Zustandsgrößen auf das Transmissionsspektrum.

Ebenfalls ist es denkbar, daß für jede der im Hinblick auf die Gasqualität zu bestimmenden physikalischen Größen mindestens ein Spektralvektor $\vec{S}$ gebildet wird, wobei die so gebildeten Spektralvektoren $\vec{S}$ Werte aus Spektralbereichen mit unterschiedlichen Grenz-Wellenlängen und/oder unterschiedlichen Größen der Spektralbereiche und/oder einer unterschiedlichen Anzahl von Spektralbereichen gebildet sein können. Je nach der oder den im Hinblick auf die Gasqualität zu bestimmenden physikalischen Größen kann es vorteilhaft sein entsprechend angepaßte Spektralvektoren $\vec{S}$ zu bestimmen, da ihrer Lage und Größe im Spektrum oder ihrer Anzahl entsprechend angepaßte Spektralbereiche eine höhere Genauigkeit der messung erlauben, als dies bei für alle im Hinblick auf die Gasqualität zu bestimmenden physikalischen Größen gleiche Spektralbereiche möglich wäre. Somit können in einer Weiterbildung für die Bildung jeder der im Hinblick auf die Gasqualität zu bestimmenden physikalischen Größen unterschiedliche Spektralbereichen herangezogen werden.

Ebenfalls ist es bei einer derartigen Anpassung der Spektralbereiche von Wichtigkeit, daß die Anzahl und Lage der Spektralbereiche von jeweils skalar miteinander multipliziertem Spektralvektor $\vec{S}$ und Faktorenvektor $\vec{V}$ einander entsprechen. Nur dann ist ein physikalisch korrektes Ergebnis zu erzielen, so daß bei der Bestimmung der Kennfelder der Faktorenvektoren $\vec{V}$ möglicherweise eine Reihe von Kennfeldern für jeweils auf anderen Spektralbereichen basierende Spektralvektoren $\vec{S}$ bestimmt werden müssen.

Eine besonders vorteilhafte Ausbildung des erfindungsgemäßen Verfahrens sieht vor, daß zur Bestimmung der im Hinblick auf die Gasqualität zu bestimmenden physikalischen Größe ein Separationsansatz durchgeführt wird, bei dem ein Anteil der zu bestimmenden physikalischen Größe im wesentlichen mit der Zusammensetzung des Probengases variiert und ein anderer Anteil im wesentlichen von dem Zustand des Probengases und nur wenig durch die Zusammensetzung des Probengases bestimmt wird. Der Anteil, der im wesentlichen von dem Zustand des Probengases bestimmt wird, kann in vorteilhafter Ausgestaltung beispielsweise aus an einem bekannten Durchschnittsgas gemessenen Zustandsdaten berechnet werden, wohingegen in weiterer Ausgestaltung der Anteil, der im wesentlichen mit der Zusammensetzung des Probengases variiert, aus dem Transmissionsspektrum des Probengases bestimmt wird. Durch diese Aufspaltung in zwei Anteile nach dem Separationsansatz läßt sich eine besondere Erhöhung der Genauigkeit bei der Bestimmung der im Hinblick auf die Gasqualität zu bestimmenden physikalischen Größen erreichen.

In einer ersten Ausgestaltung kann als im Hinblick auf die Gasqualität zu bestimmende physikalische Größe die Kompressibilitätszahl K des Probengases bestimmt werden. Eine weitere Ausgestaltung sieht vor, daß als im Hinblick auf die Gasqualität zu bestimmende physikalische Größe der Realgasfaktor Z des Probengases bestimmt wird. Ebenfalls ist denkbar, daß als im Hinblick auf die Gasqualität zu bestimmende physikalische Größe die Dichte p des Probengases und/oder der Wobbeindex $W_v$ und/oder die Methanzahl und/oder die molare Masse und oder der Taupunkt bestimmt werden.

In einer Weiterbildung ist es ebenfalls denkbar, daß als im Hinblick auf die Gasqualität zu bestimmende physikalische Größe die Dichte des Anteils von Kohlendioxid am Probengas bestimmt wird, aus der dann unter Verwendung des direkt bestimmten Realgasverhaltens, beispielsweise der Kompressibilitätszahl K oder des Realgasfaktors Z der Molenbruch $X_{CO_2}$ berechnet werden kann, also der Anteil der $CO_2$-Moleküle am Probengas. Hierdurch kann ebenfalls eine Umwertung der Betriebsdichte des Kohlendioxid auf den entsprechenden Kohlendioxid-Molanteil $X_{CO_2}$ erfolgen. Diese $CO_2$-Konzentration, die bei Verfahren der herkömmlichen Gasmeßtechnik als eine der Eingangsgrößen für die Berechnung der Kompressibilitätszahl K beispielsweise nach dem Verfahren SGERG benötigt wird, kann aus Kompatibilitätsgründen auch nach dem erfindungsgemäßen Verfahren bestimmt werden. Ebenfalls läßt sich mit dieser Erfassung der Absorption aufgrund des Kohlendioxids $CO_2$ aus dem Transmissionsspektrum eine Erhöhung der Meßgenauigkeit der anderen, im Hinblick auf die Gasqualität zu bestimmenden Größen erreichen, da sie weitere Informationen über das Gemisch der nicht brennbaren Komponenten typischer Probengase wie Stickstoff und Kohlendioxid liefert.

Von besonderem Vorteil ist es, wenn das Transmissionsspektrum des Probengases im Bereich des infraroten Lichtes, vorzugsweise im Bereich des nahen Infrarot aufgenommen wird. In diesem Spektralbereich sind die Strahlungsquellen, Detektoren und weitere optische Komponenten preiswert und in großer Anzahl sowie hoher Qualität auf dem Markt verfügbar, so daß die für die Durchführung des Verfahrens benötigte Meßtechnik kostengünstig zu erstellen ist.

Eine erste Ausgestaltung sieht vor, daß als Spektralbereich zumindest Teile des Transmissionsspektrums im Bereich zwischen etwa 1550 nm und 2050 nm genutzt werden. Eine Erfassung der Einflüsse des Probengases auf das Transmissionsspektrum in ausgewählten Abschnitten eines derart breiten Spektralbereiches erlaubt eine weitgehende Anpassung und Abstimmung des erfindungsgemäßen Verfahrens auf jeweils vorliegende Probengase, die im Rahmen der Kalibrationsmessung zur Bestimmung der Faktorenvektoren $\vec{V}$ beeinflußt werden kann.

In weiterer Ausgestaltung ist etwa als Spektralbereich zur Bestimmung des Anteiles des z. B. in Brenngasen anteilsmäßig am häufigsten vorkommenden Gases Methan $CH_4$ Strahlung bei etwa 1620 bis 1660 nm nutzbar, wobei diese und die folgenden Angaben der Spektralbereiche lediglich als bevorzugte und durch praktische Messungen besonders effektive Spektralbereiche herausgestellt werden sollen. Es versteht sich von selbst, daß bei anderen Gaszusammensetzungen oder anderen Prozeßführungen innerhalb des vorstehend genannten Bereiches andere oder weitere Spektralbereiche ausgewählt werden können. Zur Bestimmung der Anteile der Summe aller aliphatischen Kohlenwasserstoffe kann als Spektralbereich Strahlung bei etwa 1670 bis 1770 nm genutzt werden, ebenfalls ist es denkbar, daß zur Bestimmung des Anteiles des Kohlendioxides $CO_2$ Strahlung bei etwa 2000 bis 2020 nm genutzt wird. Die vorstehend genannten Werte für die Spektralbereiche liegen alle etwa im Bereich der ersten harmonischen Obertonbande der Molekülschwingungen und bieten daher besonders gute Voraussetzungen für eine korrekte Erfassung der im Hinblick auf die Gasqualität zu bestimmenden physikalischen Größen. Selbstverständlich können auch Messungen im Bereich weiterer Oberschwingungen, aber auch der Grundschwingung erfolgen.

Weiterhin ist es denkbar, daß bei der Bildung des Skalarproduktes ein Korrekturfaktor miteinbezogen werden kann, der gerätetechnische Abweichungen zwischen der Meßanordnung bei der Bestimmung der Faktorenvektoren $\vec{V}$ mit den Kalibriergasen und der Meßanordnung bei der Messung des Spektralvektors $\vec{S}$ des Probengases berücksichtigt. Ein derartiger Korrekturfaktor berücksichtigt gerätetechnische Schwankungen zwischen einer hochgenauen Kalibrations-Meßanordnung und den für den praktischen Betrieb ausgelegten Meßanordnungen, die neben einer gerätetechnischen Streuung auch gewisse Abstriche hinsichtlich der Genauigkeit machen müssen. Es wird hierzu im Rahmen einer Einmessung jeder einzelnen, für die Durchführung des erfindungsgemäßen Verfahrens bestimmten Einrichtung abgeglichen, welche Abweichungen zwischen der jeweiligen Einrichtung und der Kalibrations-Meßanordnung vorliegen, die dann als Korrekturfaktor auf die Ergebnisse der Bestimmung des Transmissionsspektrums aufgeschlagen werden. Hierdurch ist auch bei der Herstellung entsprechender Einrichtungen in Serienfertigung immer gewährleistet, daß eine serienbedingte Abweichung erfaßt und kompensiert werden kann. Bei der Kalibration unterscheidet man somit die Master- und die Exemplarkalibration:

An einem für ein Baumuster der Meßvorrichtung typischen Master-Exemplar erfolgt über ein weites Feld von wohl definierten Zuständen und einen geeigneten Satz von wohldefinierten Kalibriergasen die Messung von entsprechenden Spektralvektoren und daraus die Berechnung der entsprechenden Faktorenvektoren. Die Bestimmung der spektralen Gerätekonstante der einzelnen Serien-Exemplare des Baumusters erfolgt dann durch Messung von möglichst nur einem oder wenigen Kalibriergasen und Vergleich mit der Abweichung von der entsprechenden Masterkalibration.

Von besonderem Vorteil ist, daß als Quelle der als Probengas bei der Aufnahme des Transmissionsspektrums durchtretenden Strahlung breitbandige Strahler, vorzugsweise thermische Strahler verwendet werden, die in Weiterbildung beispielsweise unter Verwendung von Interferenzfiltern und/oder einem Monochromator und/oder optischen Detektoren das Transmissionsspektrum erfassen und meßtechnisch so aufbereiten, daß die Eingangsgrößen für das erfindungsgemäße Verfahren gewonnen werden. Ebenfalls ist es denkbar, daß als Quelle der das Probengas bei der Aufnahme des Transmissionsspektrums durchtretenden Strahlung schmalbandige Strahler, vorzugsweise LED's oder Laserlichtquellen verwendet werden, wenn ihre spektrale Charakteristik geeignet eingestellt werden kann.

Eine weitere Vereinfachung bei der Durchführung des erfindungsgemäßen Verfahrens ist es, wenn die Grenzwellenlängen der ausgewählten Spektrenbereiche bei der Aufnahme des Transmissionsspektrums dadurch bestimmt werden, daß die Grenzwellenlängen relativ zu einem Referenzsignal festgelegt werden. Bei der Aufnahme der Spektren gemäß dem vorliegenden Verfahren spielt die spektrale Lage der Banden in Bezug auf die Grenzen der gewählten Spektralbereiche eine große Rolle für die Genauigkeit der Auswertung. In üblichen Laborspektrometern als typischen Meßanordnungen wird die absolute Wellenlängengenauigkeit durch einen hohen Aufwand bei der mechanischen Ausführung und durch Verwendung von Referenzwellenlängen (Interferenzfilter, Laser) sichergestellt. Zur Vermeidung dieses hohen technischen Aufwandes für Serienmeßanordnungen kann deshalb zur Vereinfachung die absolute Wellenlängenposition anhand einer charakteristischen Lage eines Referenzsignals bestimmt und entsprechend die Wellenlängenachse korrigiert werden. Bei der Analyse von z. B. Erdgas als Probengas bietet sich hierfür in einer Weiterbildung als Referenzsignal der Bereich der höchsten Absorption der Strahlung durch das Methan, der sog. zentrale Absorptionspeak des Methans bei ca. 1666 nm an. Dieses Maximum ist besonders gut für die Korrektur geeignet, da Methan als Leitkomponente stets dominierend in einem Erdgas enthalten ist und der hohe Extinktionskoeffizient dieses Maximums für ein gutes Auffinden sorgt. Die Lage dieses Maximums wird ermittelt und relativ zu diesem Maximum die Lage der Grenzwellenlängen der ausgewählten Spektrenbereiche eingestellt. Damit lassen sich die Anforderungen an die absoluten Wellenlängengenauigkeit von entsprechenden Meßanordnungen erheblich reduzieren, der resultierende Wellenlängenfehler hängt vielmehr nur noch von der begrenzten Reproduzierbarkeit der Wellenlängeneinstellung ab. In einer Weiterbildung ist es auch denkbar, daß alternativ typische Spektrenverläufe von Stoffen als Referenzsignal genutzt werden, die etwa zusätzlich im Strahlengang der Meßanordnung zur Aufnahme des Transmissionsspektrums angeordnet sind, wie dies beispielsweise in Form von Kunststoffolien vorgenommen werden kann, die in den Referenzstrahlengang der Meßanordnung eingebracht werden.

Eine weitere Erhöhung der Genauigkeit des erfindungsgemäßen Verfahrens läßt sich dadurch erreichen, daß neben Spektralbereichen, in denen das Transmissionsspektrum des Probengases Informationen über dessen Zusammensetzung erhält, auch Spektralbereiche überwacht werden, in denen keine oder eine nur sehr geringe Absorption durch das Probengas stattfindet. Eine derartige Überwachung weiterer Spektralbereiche ist für den praktischen Einsatz der Meßanordnung in Bezug auf die Langzeitstabilität von Wichtigkeit, da es zu Variationen der Leertransmission des Meßkanals kommen kann, die nicht mit den Veränderungen des Probengases begründet sind und daher das Meßergebnis verfälschen. Die nicht oder nicht wesentlich von der Zusammensetzung des Probengases beeinflußten Spektralbereiche können daher in einer Weiterbildung zur Eigenkontrolle der Messung des Probengases in Form einer Referenz verwendet werden, wobei eine erste vorteilhafte Ausgestaltung vorsieht, daß zur Kalibration der Meßanordnung im wesentlichen zu jeder Aufnahme eines Transmissionsspektrums des Probengases oder in häufiger Abfolge eine Messung in einem absorptionsfreien Spektralbereich erfolgt. Hierzu kann beispielsweise bei der Aufnahme der Spektralbereiche stets auch ein absorptionsfreier Spektralbereich mit aufgezeichnet werden, wobei die Meßanordnung nach Aufnahme der Spektralbereiche nach dem erfindungsgemäßen Verfahren wieder eine Messung in dem nicht von dem Probengas abhängigen Spektralbereich vornimmt und die dort festgestellte Transmission mit der jeweils letzten Messung in diesem Spektralbereich vergleicht. Überschreitet eine derartige Transmissionsänderung in dem nicht von dem Probengas abhängigen Spektralbereich einen vorgebbaren Grenzwert, so kann in weiterer Ausgestaltung eine Leermessung der Meßanordnung mit einem z.B. in Erdgas absorptionsfreien Spektralbereich zum Abgleich der Meßbedingungen erfolgen. Auf diese Weise wird eine automatische Selbstkontrolle des Meßsystems durchgeführt.

Eine andere Ausgestaltung sieht vor, daß die Abweichung zu mindestens einer der vorhergehenden Messungen unterhalb des vorgebbaren Schwellwertes als Korrekturfaktor für die Messungen bei der Ermittlung der im Hinblick auf die Gasqualität zu bestimmenden physikalischen Größen berücksichtigt wird. Hierdurch kann durch Auswertung der festgestellten Abweichung eine Verbesserung der Meßergebnisse bei der eigentlichen Nutzmessung erfolgen.

Ein besonders vorteilhafter Spektralbereich für die im wesentlichen von der Zusammensetzung des Probengases unabhängigen Transmission liegt im Bereich von etwa 1500 bis 1600 nm.

Eine weitere Ausgestaltung sieht vor, daß eine oder mehrere der im Hinblick auf die Gasqualität zu bestimmenden physikalischen Größen unter Verwendung von aus dem Transmissionsspektrum unter Bildung des Skalarproduktes bestimmter Größen rein rechnerisch so bestimmt werden, daß die durch Messung und durch Berechnung ermittelten physikalischen Größen bei Einsetzen in standardisierte Näherungsverfahren das Realgasverhalten des Probengases im wesentlichen korrekt beschreiben. Wird beispielsweise zur Messung des $CO_2$-Gehaltes des Probengases etwa im Bereich oberhalb von 2000 nm das Transmissionsspektrum ausgewertet, so sind hierfür kostenaufwendige und gegebenenfalls fehleranfällige Sensoren erforderlich, die in diesem hohen Wellenlängenbereich Messungen erlauben. Derartige Sensoren kosten ein Vielfaches der jeweiligen Meßtechnik im Bereich bis etwa 1800 nm, so daß die notwendige Meßanordnung zur Durchführung des erfindungsgemäßen Verfahrens unter Einbeziehung des $CO_2$-Gehaltes deutlich gegenüber einer Meßanordnung z.B. nur zur Erfassung der Brenngase eines Probengases verteuert ist. Deswegen und auch zur Herstellung einer Kompatibilität mit Geräten der herkömmlichen Gasmeßtechnik kann daher der Gehalt an Kohlendioxid im Probengas rein rechnerisch dadurch bestimmt werden, daß anhand des erfindungsgemäßen Verfahrens das Realgasverhalten sowie weitere im Hinblick auf die Gasqualität zu bestimmenden physikalischen Größen aus dem Skalarprodukt von Spektralvektor $\vec{S}$ und Faktorenvektor $\vec{V}$ bestimmt werden. Die meßtechnisch nicht erfaßte Komponente, beispielsweise also der Kohlendioxidanteil, kann dann aus den wie vorstehend bestimmten Größen so berechnet werden, daß sich etwa durch Umkehrung eines standardisierten Verfahrens wie z.B. dem Verfahren SGERG oder dgl. nach Anwendung der entsprechenden Verfahren wieder das richtige Realgasverhalten ergibt. Es handelt sich hierbei um eine für viele Fälle der Anwendung des erfindungsgemäßen Verfahrens ausreichend genaue Bestimmung etwa des Gehaltes an Kohlendioxid, ohne daß das Verfahren aufgrund seiner gerätetechnischen Umsetzung besonders verkompliziert bzw. verteuert wird.

**Patentansprüche**

1. Verfahren zur Bestimmung der Gasqualität eines Probengases, insbesondere eines Brenngases, ausgehend von einem unter Betriebsbedingungen mittels spektroskopischer Meßverfahren bestimmten Transmissionsspektrums des Probengases,
wobei

- in einer Zustandsmessung zumindest die zugehörigen Werte des Druckes p und der Temperatur T des Probengases ermittelt werden,
- in einer Anzahl von ausgewählten Spektralbereichen einer Bande durch Integration von Werten des Transmissionsspektrums des Probengases mindestens ein Spektralvektor $\bar{S}$ gebildet wird, der als Komponenten die Werte der Integrale über die ausgewählten Spektralbereiche aufweist und charakteristisch für Eigenschaften des Probengases im Betriebszustand ist,

- zur Bestimmung einer im Hinblick auf die Gasqualität zu bestimmenden physikalischen Größe der Spektralvektor $\bar{S}$ skalar mit einem Faktorenvektor $\vec{V}$ multipliziert wird, der durch Kalibrationsmessungen von Spektralvektoren $\bar{S}$ an Kalibriergasen bekannter Eigenschaften und unter bekannten Zustandsbedingungen ermittelt wurde, wobei für jede der im Hinblick auf die Gasqualität zu bestimmenden physikalischen Größen aus einem Kennfeld für einen Zustand des Probengases ein zugehöriger Fraktorenvektor $\vec{V}$ ermittelt wird

und je nach der im Hinblick auf die Gasqualität zu bestimmenden physikalischen Größe ein zugehöriger Faktorenvektor $\vec{V}$ herangezogen wird.

2. Verfahren nach Anspruch 1, **dadurch gekennzeichnet, daß** bei den Kalibrationsmessungen die Faktorenvektoren $\vec{V}$ in Form eines Kennfeldes unter Variation von Druck p und Temperatur T der verwendeten Kalibriergase bestimmt werden.

3. Verfahren nach einem der Ansprüche 1 oder 2, **dadurch gekennzeichnet, daß** zur Bildung des Skalarproduktes mit dem jeweilig zugehörigen Spektralvektor $\bar{S}$ derjenige Faktorenvektor $\vec{V}$ aus dem Kennfeld ausgewählt wird, der den Zustandsbedingungen des Probengases bei der Aufnahme des Spektrums des Probengases und der im Hinblick auf die Gasqualität zu bestimmenden physikalischen Größe entspricht.

4. Verfahren nach einem der Ansprüche 1 bis 3, **dadurch gekennzeichnet, daß** für jede der im Hinblick auf die Gasqualität zu bestimmenden physikalischen Größen mindestens ein Spektralvektor $\bar{S}$ gebildet wird, wobei die so gebildeten Spektralvektoren $\bar{S}$ Werte aus Spektralbereichen mit unterschiedlichen Grenz-Wellenlängen und/oder unterschiedlichen Größen der Spektralbereiche und/oder einer unterschiedlichen Anzahl von Spektralbereichen gebildet sein können.

5. Verfahren nach Anspruch 4, **dadurch gekennzeichnet, daß** für die Bildung jeder der im Hinblick auf die Gasqualität zu bestimmenden physikalischen Größen unterschiedliche Spektralbereiche herangezogen werden.

6. Verfahren nach einem der vorstehenden Ansprüche, **dadurch gekennzeichnet, daß** die Anzahl und Lage der Spektralbereiche von jeweils skalar miteinander multipliziertem Spektralvektor $\bar{S}$ und Faktorenvektor $\vec{V}$ einander entsprechen.

7. Verfahren nach einem der vorstehenden Ansprüche, **dadurch gekennzeichnet, daß** zur Bestimmung der im Hinblick auf die Gasqualität zu bestimmenden physikalischen Größe ein Separationsansatz durchgeführt wird, bei dem ein Anteil der zu bestimmenden physikalischen Größe im wesentlichen mit der Zusammensetzung des Probengases variiert und ein anderer Anteil im wesentlichen von dem Zustand des Probengases und durch die Zusammensetzung des Probengases nur wenig bestimmt wird.

8. Verfahren nach Anspruch 7, **dadurch gekennzeichnet, daß** der Anteil, der im wesentlichen von dem Zustand des Probengases bestimmt wird, aus an einem bekannten Durchschnittsgas gemessenen Zustandsdaten berechnet wird.

9. Verfahren nach einem der Ansprüche 7 oder 8, **dadurch gekennzeichnet, daß** der Anteil, der im wesentlichen mit der Zusammensetzung des Probengases variiert, aus dem Transmissionsspektrum des Probengases bestimmt wird.

10. Verfahren nach einem der vorstehenden Ansprüche, **dadurch gekennzeichnet, daß** als im Hinblick auf die Gasqualität zu bestimmende physikalische Größe die Kompressibilitätszahl K des Probengases bestimmt wird.

11. Verfahren nach einem der vorstehenden Ansprüche, **dadurch gekennzeichnet, daß** als im Hinblick auf die Gasqualität zu bestimmende physikalische Größe der Realgasfaktor Z des Probengases bestimmt wird.

12. Verfahren nach einem der vorstehenden Ansprüche, **dadurch gekennzeichnet, daß** als im Hinblick auf die Gasqualität zu bestimmende physikalische Größe die Dichte $\rho$ des Probengases bestimmt wird.

13. Verfahren nach einem der vorstehenden Ansprüche, **dadurch gekennzeichnet, daß** als im Hinblick auf die Gasqualität zu bestimmende physikalische Größe der Wobbe-Index des Probengases bestimmt wird.

14. Verfahren nach einem der vorstehenden Ansprüche, **dadurch gekennzeichnet, daß** als im Hinblick auf die Gasqualität zu bestimmende physikalische Größe die Methanzahl des Probengases bestimmt wird.

15. Verfahren nach einem der vorstehenden Ansprüche, **dadurch gekennzeichnet, daß** als im Hinblick auf die Gasqualität zu bestimmende physikalische Größe die Molare Masse des Probengases bestimmt wird.

16. Verfahren nach einem der vorstehenden Ansprüche, **dadurch gekennzeichnet, daß** als im Hinblick auf die Gasqualität zu bestimmende physikalische Größe die Dichte des Anteils von Kohlendioxid am Probengas bestimmt wird.

17. Verfahren nach einem der vorstehenden Ansprüche, **dadurch gekennzeichnet, daß** als im Hinblick auf die Gasqualität zu bestimmende physikalische Größe der Taupunkt des Probengases bestimmt wird.

18. Verfahren nach einem der vorstehenden Ansprüche, **dadurch gekennzeichnet, daß** das Transmissionsspektrum des Probengases im Bereich des infraroten Lichtes, vorzugsweise im Bereich des nahen Infrarot aufgenommen wird.

19. Verfahren nach Anspruch 18, **dadurch gekennzeichnet, daß** als Spektralbereiche zumindest Teile des Transmissionsspektrums im Bereich zwischen etwa 1550 nm und 2050 nm genutzt werden.

20. Verfahren nach Anspruch 19, **dadurch gekennzeichnet, daß** als Spektralbereich zur Bestimmung des Anteiles des Methan $CH_4$ Strahlung bei etwa 1620 bis 1660 nm genutzt wird.

21. Verfahren nach Anspruch 19, **dadurch gekennzeichnet, daß** als Spektralbereich zur Bestimmung des Anteiles der Summe aller aliphatischen Kohlenwasserstoffe Strahlung bei etwa 1670 bis 1770 nm genutzt wird.

22. Verfahren nach Anspruch 19, **dadurch gekennzeichnet, daß** als Spektralbereich zur Bestimmung des Anteiles des Kohlendioxids $CO_2$ Strahlung bei etwa 2000 bis 2020 nm genutzt wird.

23. Verfahren nach einem der vorstehenden Ansprüche, **dadurch gekennzeichnet, daß** bei der Bildung des Skalarproduktes eine Korrekturfunktion mit einbezogen wird, die gerätetechnische Abweichungen zwischen der Meßanordnung bei der Bestimmung der Faktorenvektoren $\vec{V}$ mit den Kalibriergasen und der Meßanordnung bei der Messung des Spektralvektors $\vec{S}$ des Probengases berücksichtigt.

24. Verfahren nach Anspruch 23, **dadurch gekennzeichnet, daß** zur Bildung der Korrekturfunktion die Meßanordnung für die Messung des Spektralvektors $\vec{S}$ des Probengases einmalig eingemessen und diese Ergebnisse in Verhältnis zu entsprechenden Ergebnissen der Meßanordnung bei der Aufnahme der Faktorenvektoren $\vec{V}$ mit den Kalibriergasen gesetzt wird.

25. Verfahren nach einem der vorstehenden Ansprüche, **dadurch gekennzeichnet, daß** als Quelle der das Probengas bei der Aufnahme des Transmissionsspektrums durchtretenden Strahlung breitbandige Strahler, vorzugsweise thermische Strahler verwendet werden.

26. Verfahren nach Anspruch 25, **dadurch gekennzeichnet, daß** die Aufnahme des Transmissionsspektrums unter Verwendung von Interferenzfiltern und/oder einem Monochromator und/oder optischen Detektoren erfolgt.

27. Verfahren nach einem der Ansprüche 1 bis 24, **dadurch gekennzeichnet, daß** als Quelle der das Probengas bei der Aufnahme des Transmissionsspektrums durchtretenden Strahlung schmalbandige Strahler, vorzugsweise LED's oder Laserlichtquellen verwendet werden.

28. Verfahren nach einem der vorstehenden Ansprüche, **dadurch gekennzeichnet, daß** die Grenzwellenlängen der ausgewählten Spektrenbereiche bei der Aufnahme des Transmissionsspektrums **dadurch** bestimmt werden, daß die Grenzwellenlängen relativ zu einem Referenzsignal festgelegt werden.

29. Verfahren nach Anspruch 28, **dadurch gekennzeichnet, daß** als Referenzsignal eine charakteristische Signatur des aufzunehmenden Transmissionsspektrums, vorzugsweise der Bereich der höchsten Absorption der Strahlung durch Methan, genutzt wird.

30. Verfahren nach Anspruch 28, **dadurch gekennzeichnet, daß** als Referenzsignal typische Spektrenverläufe von Stoffen genutzt werden, die zusätzlich im Strahlengang der Anordnung zur Messung des Transmissionsspektrums angeordnet werden.

31. Verfahren nach einem der vorstehenden Ansprüche, **dadurch gekennzeichnet, daß** neben Spektralbereichen, in denen das Transmissionsspektrum des Probengases Informationen über dessen Zusammensetzung enthält, auch Spektralbereiche überwacht werden, in denen keine oder nur eine sehr geringe Absorption durch das Probengas stattfindet.

32. Verfahren nach Anspruch 31, **dadurch gekennzeichnet, daß** die ganz oder nahezu absorptionsfreien Spektralbereiche zur Eigenkontrolle der Messung des Probengases in Form einer Referenz verwandt werden.

33. Verfahren nach einem der Ansprüche 31 oder 32, **dadurch gekennzeichnet, daß** zur Kalibration der Meßanordnung im wesentlichen zu jeder Aufnahme eines Transmissionsspektrums des Probengases oder in häufiger Abfolge eine Messung in einem absorptionsfreien Spektralbereich des Probengases erfolgt.

34. Verfahren nach einem der Ansprüche 31 bis 33, **dadurch gekennzeichnet, daß** eine Leermessung der Meßanordnung mit einem Inertgas zum Abgleich der Meßbedingungen immer erst dann vorgenommen wird, wenn bei der Messung in den ganz oder nahezu absorptionsfreien Spektralbereichen Abweichungen zu mindestens einer der vorhergehenden Messungen in diesem absorptionsfreien Spektralbereich auftreten.

35. Verfahren nach Anspruch 34, **dadurch gekennzeichnet, daß** eine Leermessung der Meßanordnung mit einem Inertgas zum Abgleich der Meßbedingungen immer erst dann vorgenommen wird, wenn die Messung in den ganz oder nahezu absorptionsfreien Spektralbereichen die Abweichung zu mindestens einer der vorhergehenden Messungen einen vorgebbaren Schwellwert überschreitet.

36. Verfahren nach Anspruch 34, **dadurch gekennzeichnet, daß** die Abweichung zu mindestens einer der vorhergehenden Messungen unterhalb des vorgebbaren Schwellwertes als Korrekturfaktor für die Messungen bei der Ermittlung der im Hinblick auf die Gasqualität zu bestimmenden physikalischen Größen berücksichtigt wird.

37. Verfahren nach einem der Ansprüche 31 bis 36, **dadurch gekennzeichnet, daß** die ganz oder nahezu absorptionsfreien Spektralbereiche im Bereich von etwa 1500 bis 1600 nm liegen.

38. Verfahren nach einem der vorstehenden Ansprüche, **dadurch gekennzeichnet, daß** eine oder mehrere der im Hinblick auf die Gasqualität zu bestimmenden physikalischen Größen unter Verwendung von aus dem Transmissionsspektrum unter Bildung des Skalarproduktes bestimmter Größen rein rechnerisch so bestimmt werden, daß die durch Messung und durch Berechnung ermittelten physikalischen Größen bei Einsetzen in standardisierte Näherungsverfahren das Realgasverhalten des Probengases im wesentlichen korrekt beschreiben.

**Claims**

1. A method of determining the gas quality of a sample gas, in particular a fuel gas, based on a transmission spectrum of the sample gas which is determined under operating conditions by means of spectroscopic measurement methods, wherein

    - in a state measurement operation at least the associated values of the pressure p and the temperature T of the sample gas are determined,
    - at least one spectral vector $\overline{S}$ is formed in a number of selected spectral regions of a band by integration of values of the transmission spectrum of the sample gas, which spectral vector has as components the values of the integrals over the selected spectral regions and is characteristic for properties of the sample gas in the operating condition,
    - for determining a physical parameter which is to be determined in relation to the gas quality the spectral vector $\overline{S}$ is scalarly multiplied by a factor vector $\overline{V}$ which was ascertained by calibration measurements of spectral vectors $\overline{S}$ on calibration gases of known properties and under known state conditions, wherein for each of the physical parameters to be determined in relation to the gas quality an associated factor vector $\overline{V}$ is ascertained from a characteristic field for a state of the sample gas,
    - and depending on the respective physical parameter to be determined in relation to the gas quality an associated factor vector $\overline{V}$ is used.

2. A method according to claim 1 **characterised in that** in the calibration measurement operations the factor vectors $\overline{V}$ are determined in the form of a characteristic field with a variation in the pressure p and the temperature T of the calibrating gases used.

3. A method according to one of claims 1 and 2 **characterised in that** to form the scalar product with the respectively associated spectral vector $\overline{S}$ that factor vector $\overline{V}$ is selected from the characteristic field, which corresponds to the state conditions of the sample gas when recording the spectrum of the sample gas and the physical parameter to be determined in relation to the gas quality.

4. A method according to one of claims 1 to 3 **characterised in that** at least one spectral vector $\overline{S}$ is formed for each of the physical parameters to be determined in relation to the gas quality, wherein the spectral vectors $\overline{S}$ formed **in that** way can be values from spectral regions with different limit wavelengths and/or different values in respect of the spectral regions and/or a different number of spectral regions.

5. A method according to claim 4 **characterised in that** different spectral regions are used for forming each physical parameter to be determined in relation to the gas quality.

6. A method according to one of the preceding claims **characterised in that** the number and position of the spectral regions of respective scalarly mutually multiplied spectral vector $\overline{S}$ and factor vector $\overline{V}$ correspond to each other.

7. A method according to one of the preceding claims **characterised in that** a separation method is implemented for determining the physical parameter to be determined in relation to the gas quality, in which a proportion of the physical parameter to be determined is varied substantially with the composition of the sample gas and another proportion is only little determined substantially by the state of the sample gas and by the composition of the sample gas.

8. A method according to claim 7 **characterised in that** the proportion which is determined substantially by the state of the sample gas is calculated from state data measured on a known average gas.

9. A method according to one of claims 7 and 8 **characterised in that** the proportion which varies substantially with the composition of the sample gas is determined from the transmission spectrum of the sample gas.

10. A method according to one of the preceding claims **characterised in that** the compressibility coefficient K of the sample gas is determined as the physical parameter to be determined in relation to the gas quality.

11. A method according to one of the preceding claims **characterised in that** the real gas factor Z of the sample gas is determined as the physical parameter to be determined in relation to the gas quality.

12. A method according to one of the preceding claims **characterised in that** the density ρ of the sample gas is determined as the physical parameter to be determined in relation to the gas quality.

13. A method according to one of the preceding claims **characterised in that** the Wobbe index of the sample gas is determined as the physical parameter to be determined in relation to the gas quality.

14. A method according to one of the preceding claims **characterised in that** the methane factor of the sample gas is determined as the physical parameter to be determined in relation to the gas quality.

15. A method according to one of the preceding claims **characterised in that** the molar mass of the sample gas is determined as the physical parameter to be determined in relation to the gas quality.

16. A method according to one of the preceding claims **characterised in that** the density of the proportion of carbon dioxide in the sample gas is determined as the physical parameter to be determined in relation to the gas quality.

17. A method according to one of the preceding claims **characterised in that** the dew point of the sample gas is determined as the physical parameter to be determined in relation to the gas quality.

18. A method according to one of the preceding claims **characterised in that** the transmission spectrum of the sample gas is recorded in the range of infrared light, preferably in the range of near infrared.

19. A method according to claim 18 **characterised in that** at least parts of the transmission spectrum in the range between about 1550 nm and 2050 nm are used as spectral regions.

20. A method according to claim 19 **characterised in that** radiation at about 1620 to 1660 nm is used as the spectral region for determining the proportion of methane $CH_4$.

21. A method according to claim 19 **characterised in that** radiation at about 1670 to 1770 nm is used as the spectral region for determining the proportion of the total of all aliphatic hydrocarbons.

22. A method according to claim 19 **characterised in that** radiation at about 2000 to 2020 nm is used as the spectral region for determining the proportion of carbon dioxide $CO_2$.

23. A method according to one of the preceding claims **characterised in that** in the formation of the scalar product a correction function is incorporated, which takes account of apparatus differences between the measurement arrangement when determining the factor vectors V with the calibration gases and the measurement arrangement in the measurement of the spectral vector $\overline{S}$ of the sample gas.

24. A method according to claim 23 **characterised in that** to form the correction function the measurement arrangement for measuring the spectral vector $\overline{S}$ of the sample gas is measured once and those results are related to corresponding results of the measurement arrangement when recording the factor vectors $\overline{V}$ with the calibration gases.

25. A method according to one of the preceding claims **characterised in that** wide-band radiating devices, preferably thermal radiating devices, are used as the source of the radiation which passes through the sample gas in recording the transmission spectrum.

26. A method according to claim 25 **characterised in that** recording of the transmission spectrum is effected using interference filters and/or a monochromator and/or optical detectors.

27. A method according to one of claims 1 to 24 **characterised in that** narrow-band radiating devices, preferably LEDs or laser light sources, are used as the source of the radiation which passes through the sample gas in recording the transmission spectrum.

28. A method according to one of the preceding claims **characterised in that** the limit wavelengths of the selected spectral regions when recording the transmission spectrum are determined by the limit wavelengths being established relative to a reference signal.

29. A method according to claim 28 **characterised in that** a characteristic signature of the transmission spectrum to be recorded, preferably the region of the highest absorption of the radiation by methane, is used as the reference signal.

**30.** A method according to claim 28 **characterised in that** typical spectral configurations of substances which are additionally arranged in the beam path of the arrangement for measuring the transmission spectrum are used as the reference signal.

**31.** A method according to one of the preceding claims **characterised in that**, besides spectral regions in which the transmission spectrum of the sample gas contains information about the composition thereof, spectral regions are also monitored in which no or only a very low level of absorption by the sample gas occurs.

**32.** A method according to claim 31 **characterised in that** the entirely or almost absorption-free spectral regions are used for self-control of the measurement of the sample gas in the form of a reference.

**33.** A method according to one of claims 31 and 32 **characterised in that** a measurement operation is effected in an absorption-free spectral region of the sample gas for calibration of the measurement arrangement substantially at each recording of a transmission spectrum of the sample gas or in frequent succession.

**34.** A method according to one of claims 31 to 33 **characterised in that** empty measurement of the measurement arrangement is effected with an inert gas for adjustment of the measurement conditions only whenever in measurement in the entirely or almost absorption-free spectral regions differences occur in relation to at least one of the preceding measurements in said absorption-free spectral region.

**35.** A method according to claim 34 **characterised in that** empty measurement of the measurement arrangement is effected with an inert gas for adjustment of the measurement conditions only whenever in measurement in the entirely or almost absorption-free spectral regions the difference in relation to at least one of the preceding measurements exceeds a predeterminable reference value.

**36.** A method according to claim 34 **characterised in that** the difference in relation to at least one of the preceding measurements below the predeterminable threshold value is taken into account as a correction factor for the measurements in ascertaining the physical parameters to be determined in relation to the gas quality.

**37.** A method according to one of claims 31 to 36 **characterised in that** the entirely or almost absorption-free spectral regions are in the region of about 1500 to 1600 nm.

**38.** A method according to one of the preceding claims **characterised in that** one or more of the physical parameters to be determined in relation to the gas quality are determined purely by calculation using parameters determined from the transmission spectrum with the formation of the scalar product, in such a way that the physical parameters determined by measurement and by calculation substantially correctly describe the real gas behaviour of the sample gas when introduced into standardised approximation methods.

**Revendications**

**1.** Procédé pour déterminer la qualité de gaz d'un gaz d'échantillon, en particulier d'un gaz de combustion, partant d'un spectre de transmission, déterminé dans des conditions de service au moyen de procédés de mesure de spectroscopie du gaz d'échantillon,

   - les valeurs spécifiques de la pression p et de la température T du gaz d'échantillon étant déterminées dans une mesure d'état,
   - au moins un vecteur spectral S étant formé dans un certain nombre de plages spectrales sélectionnées d'une bande par intégration de valeurs du spectre de transmission du gaz d'échantillon, lequel vecteur présente comme composants les valeurs de l'intégrale par rapport aux plages spectrales sélectionnées et est caractéristique des propriétés du gaz d'échantillon dans l'état de service,
   - pour déterminer une grandeur physique à déterminer par rapport à la qualité du gaz, le vecteur spectral étant multiplié de façon scalaire par un vecteur de facteurs V, qui a été déterminé par des mesures d'étalonnage de vecteurs spectraux S sur des gaz d'étalonnage de propriétés connues et dans des conditions d'état connues, un vecteur de facteurs V spécifique étant déterminé pour chacune des grandeurs physiques à déterminer par rapport à la qualité du gaz à partir d'un champ caractéristique pour un état du gaz d'échantillon et un vecteur de facteurs V spécifique étant utilisé selon la grandeur physique à déterminer par rapport à la qualité du gaz.

**2.** Procédé selon la revendication 1, **caractérisé en ce que**, lors des mesures d'étalonnage, les vecteurs de facteurs V sont déterminés sous la forme d'un champ caractéristique en faisant varier la pression p et la température T des gaz d'étalonnage utilisés.

**3.** Procédé selon l'une quelconque des revendications 1 ou 2, **caractérisé en ce que**, pour former le produit scalaire avec le vecteurs spectral S spécifique, on choisit le vecteur de facteur V dans le champ caractéristique qui correspond aux conditions d'état du gaz d'échantillon lors de l'enregistrement du spectre du gaz d'échantillon et de la grandeur physique à déterminer par rapport à la qualité du gaz.

**4.** Procédé selon l'une quelconque des revendications 1 à 3, **caractérisé en ce que**, pour chacune des grandeurs physiques à déterminer par rapport à la qualité du gaz, on forme au moins un vecteur spectral S, les vecteurs spectraux S valeurs ainsi formés pouvant être formés à partir de plages spectrales avec différentes longueurs d'ondes limites et/ou différentes grandeurs des plages spectrales et/ou un nombre différent de plages spectrales.

**5.** Procédé selon la revendication 4, **caractérisé en ce que** différentes plages spectrales sont utilisées pour la formation de chacune des grandeurs physiques à déterminer par rapport à la qualité du gaz.

**6.** Procédé selon l'une quelconque des revendications précédentes, **caractérisé en ce que** le nombre et la position des plages spectrales du vecteur spectral S et du vecteur des facteurs V multipliés à chaque fois de façon scalaire entre eux se correspondent.

**7.** Procédé selon l'une quelconque des revendications précédentes, **caractérisé en ce que**, pour déterminer la grandeur physique à déterminer par rapport à la qualité du gaz, on effectue un début de séparation, dans lequel une partie de la grandeur physique à déterminer varie essentiellement avec la composition du gaz d'échantillon et une autre fraction n'est que peu déterminée principalement par l'état du gaz d'échantillon et par la composition du gaz d'échantillon.

**8.** Procédé selon la revendication 7, **caractérisé en ce que** la fraction, qui est déterminée essentiellement par l'état du gaz d'échantillon, est calculée à partir de données d'état mesurées sur un gaz moyen connu.

**9.** Procédé selon l'une quelconque des revendications 7 ou 8, **caractérisé en ce que** la fraction, qui varie essentiellement avec la composition du gaz d'échantillon, est déterminée à partir du spectre de transmission du gaz d'échantillon.

**10.** Procédé selon l'une quelconque des revendications précédentes, **caractérisé en ce que** l'indice de compressibilité K du gaz d'échantillon est déterminé en tant que grandeur physique à déterminer par rapport à la qualité du gaz.

**11.** Procédé selon l'une quelconque des revendications précédentes, **caractérisé en ce que** le facteur du gaz réel Z du gaz d'échantillon est déterminé en tant que grandeur physique à déterminer par rapport à la qualité du gaz.

**12.** Procédé selon l'une quelconque des revendications précédentes, **caractérisé en ce que** la densité p du gaz d'échantillon est déterminée en tant que grandeur physique à déterminer par rapport à la qualité du gaz.

**13.** Procédé selon l'une quelconque des revendications précédentes, **caractérisé en ce que** l'indice de Wobbe du gaz d'échantillon est déterminé en tant que grandeur physique à déterminer par rapport à la qualité du gaz.

**14.** Procédé selon l'une quelconque des revendications précédentes, **caractérisé en ce que** l'indice de méthane du gaz d'échantillon est déterminé en tant que grandeur physique à déterminer par rapport à la qualité du gaz.

**15.** Procédé selon l'une quelconque des revendications précédentes, **caractérisé en ce que** la masse molaire du gaz d'échantillon est déterminée en tant que grandeur physique à déterminer par rapport à la qualité du gaz.

**16.** Procédé selon l'une quelconque des revendications précédentes, **caractérisé en ce que** la densité de la fraction de dioxyde de carbone dans le gaz d'échantillon est déterminée en tant que grandeur physique à déterminer par rapport à la qualité du gaz.

**17.** Procédé selon l'une quelconque des revendications précédentes, **caractérisé en ce que** le point de rosée du gaz d'échantillon est déterminé en tant que grandeur physique à déterminer par rapport à la qualité du gaz.

**18.** Procédé selon l'une quelconque des revendications précédentes, **caractérisé en ce que** le spectre de transmission du gaz d'échantillon est enregistré dans la plage de la lumière infrarouge, de préférence dans la plage de l'infrarouge proche.

**19.** Procédé selon la revendication 18, **caractérisé en ce que**, comme plages spectrales, on utilise au moins des parties du spectre de transmission dans la plage comprise entre environ 1 550 nm et 2 050 nm.

**20.** Procédé selon la revendication 19, **caractérisé en ce que**, comme plage spectrale pour déterminer la fraction du méthane $CH_4$, on utilise du rayonnement proche de 1 620 jusqu'à 1 660 nm.

**21.** Procédé selon la revendication 19, **caractérisé en ce que**, comme plage spectrale pour déterminer la fraction de la somme de tous les hydrocarbures aliphatiques, on utilise du rayonnement proche de 1 670 jusqu'à 1 770 nm.

**22.** Procédé selon la revendication 19, **caractérisé en ce que**, comme plage spectrale pour déterminer la fraction du dioxyde de carbone $CO_2$, on utilise du rayonnement proche de 2 000 jusqu'à 2 020 nm.

**23.** Procédé selon l'une quelconque des revendications précédentes, **caractérisé en ce que**, lors la formation du produit scalaire, on intègre une fonction de correction qui prend en compte les écarts d'appareillage entre le dispositif de mesure pour le calcul des vecteurs de facteurs V avec les gaz d'étalonnage et le dispositif de mesure pour la mesure du vecteur spectral S du gaz d'échantillon.

**24.** Procédé selon la revendication 23, **caractérisé en ce que**, pour former la fonction de correction, le dispositif de mesure pour la mesure du vecteur spectral S du gaz d'échantillon est intégré une fois dans la mesure et ces résultats sont mis en rapport avec des résultats correspondants du dispositif de mesure lors de l'enregistrement des vecteurs de facteurs V avec les gaz d'étalonnage.

**25.** Procédé selon l'une quelconque des revendications précédentes, **caractérisé en ce que**, comme source du rayonnement traversant le gaz d'échantillon lors de l'enregistrement du spectre de transmission, on utilise des sources de rayonnement à large bande, de préférence des sources de rayonnement thermique.

**26.** Procédé selon la revendication 25, **caractérisé en ce que** l'enregistrement du spectre de transmission s'effectue en utilisant des filtres d'interférence et/ou un monochromateur et/ou des détecteurs optiques.

**27.** Procédé selon l'une quelconque des revendications 1 à 24, **caractérisé en ce que** des sources de rayonnement à bande étroite, de préférence des LEDs ou des sources de lumière laser, sont utilisées comme source du rayonnement traversant le gaz d'échantillon lors de l'enregistrement du spectre de transmission.

**28.** Procédé selon l'une quelconque des revendications précédentes, **caractérisé en ce que** les longueurs d'ondes limites des plages de spectres sélectionnées sont déterminées lors de l'enregistrement du spectre de transmission par le fait que les longueurs d'ondes limites sont définies par rapport à un signal de référence.

**29.** Procédé selon la revendication 28, **caractérisé en ce que**, comme signal de référence, on utilise une signature caractéristique du spectre de transmission à enregistrer, de préférence la plage de l'absorption maximale du rayonnement par du méthane.

**30.** Procédé selon la revendication 28, **caractérisé en ce que**, comme signal de référence, on utilise des courbes de spectres caractéristiques de matières qui sont disposées en supplément dans le trajet des faisceaux du dispositif destiné à mesurer le spectre de transmission.

**31.** Procédé selon l'une quelconque des revendications précédentes, **caractérisé en ce que**, parallèlement à des plages spectrales dans lesquelles le spectre de transmission du gaz d'échantillon contient des informations sur sa composition, on contrôle également des plages spectrales dans lesquelles aucune absorption ou seulement une absorption très faible par le gaz d'échantillon a lieu.

**32.** Procédé selon la revendication 31, **caractérisé en ce que** les plages spectrales complètement exemptes d'absorption ou pratiquement sans absorption sont utilisées pour l'autocontrôle de la mesure du gaz d'échantillon sous la forme d'une référence.

**33.** Procédé selon l'une quelconque des revendications 31 ou 32, **caractérisé en ce que**, pour l'étalonnage du dispositif de mesure, on procède à une mesure dans une plage spectrale sans absorption du gaz d'échantillon sensiblement pour chaque enregistrement d'un spectre de transmission du gaz d'échantillon ou dans une succession fréquente.

**34.** Procédé selon l'une quelconque des revendications 31 à 33, **caractérisé en ce qu'**une mesure à vide du dispositif de mesure avec un gaz inerte pour l'ajustement des conditions de mesure est toujours effectuée uniquement dans le cas où, lors de la mesure, il apparaît dans les plages spectrales sans aucune absorption ou pratiquement sans absorption des écarts par rapport à au moins l'une des mesures antérieures dans cette plage spectrale sans absorption.

**35.** Procédé selon la revendication 34, **caractérisé en ce qu'**une mesure à vide du dispositif de mesure avec un gaz inerte pour l'ajustement des conditions de mesure est toujours effectuée seulement dans le cas où, lors de la mesure, dans les plages spectrales sans aucune absorption ou pratiquement sans absorption, l'écart par rapport à au moins l'une des mesures précédentes dépasse une valeur seuil prédéfinissable.

**36.** Procédé selon la revendication 34, **caractérisé en ce que** l'écart par rapport à au moins l'une des mesures antérieures au-dessous de la valeur seuil prédéfinissable est pris en compte en tant que facteur de correction pour les mesures lors du calcul des grandeurs physiques à déterminer par rapport à la qualité du gaz.

**37.** Procédé selon l'une quelconque des revendications 31 à 36, **caractérisé en ce que** les plages spectrales exemptes d'absorption ou pratiquement sans absorption se situent dans la plage d'environ 1 500 à 1 600 nm.

**38.** Procédé selon l'une quelconque des revendications précédentes, **caractérisé en ce qu'**une ou plusieurs des grandeurs physiques à déterminer par rapport à la qualité du gaz sont déterminées de façon purement mathématique en utilisant des grandeurs déterminées à partir du spectre de transmission formant le produit scalaire, de telle sorte que les grandeurs physiques déterminées par la mesure et par le calcul décrivent sensiblement correctement le comportement du gaz réel du gaz d'échantillon si elles sont utilisées dans des procédés d'approximation standardisés.